(19) Europäisches Patentamt
European Patent Office
Office européen des brevets

(11) EP 1 551 471 B1

(12) EUROPEAN PATENT SPECIFICATION

(45) Date of publication and mention of the grant of the patent:
**01.04.2009 Bulletin 2009/14**

(21) Application number: **03771809.5**

(22) Date of filing: **25.07.2003**

(51) Int Cl.:
***A61L 27/34*** *(2006.01)* ***C08L 89/04*** *(2006.01)*

(86) International application number:
**PCT/US2003/023207**

(87) International publication number:
**WO 2004/011052 (05.02.2004 Gazette 2004/06)**

(54) **BIOACTIVE COATING FOR MEDICAL DEVICES COMPRISING KERATIN**

BIOAKTIVE BESCHICHTUNG FÜR MEDIZINISCHE VORRICHTUNG MIT KERATIN

REVETEMENT BIOACTIF POUR DISPOSITIFS MEDICAUX COMPRENANT DE LA KERATINE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **25.07.2002 US 399039 P**

(43) Date of publication of application:
**13.07.2005 Bulletin 2005/28**

(73) Proprietor: **Keraplast Technologies Ltd.**
**San Antonio, TX 78258 (US)**

(72) Inventors:
- **VAN DYKE, Mark**
**Fair Oaks Ranch, TX 78015 (US)**
- **SILLER-JACKSON, Arlene, J.**
**Helotes, TX 78023 (US)**

(74) Representative: **Glawe, Delfs, Moll**
**Patentanwälte**
**Rothenbaumchaussee 58**
**20148 Hamburg (DE)**

(56) References cited:
**WO-A-03/008006**
**US-A- 5 356 433**
**US-A- 5 358 935**
**US-A- 6 159 495**

- **VAN DYKE MARK E ET AL: "Development of keratin coatings for osteoinduction on titanium" ABSTRACTS OF PAPERS AMERICAN CHEMICAL SOCIETY, vol. 224, no. 1-2, 2002, page PMSE 100 XP009021995 224th National Meeting of the American Chemical Society;Boston, MA, USA; August 18-22, 2002 ISSN: 0065-7727**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present application relates to coatings for medical devices, preferably coatings for medical implants. More particularly, the application relates to bioactive coatings, preferably bioactive coatings comprising keratin having osteoinductive properties.

### Background of the Invention

Many different types of coatings have been developed for purposes of passivating the surface of a medical device. Collagen, hydroxyapatite, and more recently the so-called RGD peptides all have been investigated as coatings in orthopedic implant applications. These materials all have been shown to be at least osteoconductive, but none have been shown to be osteoinductive. Passivating coatings are needed which have osteoinductive properties and which can mask the implant from the human immune system.

### Brief Summary

The present application provides a medical implant comprising a passivating coating comprising keratin. The passivating coating comprising a bonding region and a bioactive region. The said bonding region comprises at least one organosilane compound comprising a silane component bound to a surface of said substrate. The bioactive region comprises an organic component of said organosilane bound to a reactive pendant group on the keratin.

### Detailed Summary

The present application provides substrates comprising a passivating coating comprising keratin. In a preferred embodiment, the passivating coating comprises osteoinductive properties. In a more preferred embodiment, the passivating coating comprises both osteoinductive properties and osteoconductive properties.

The substrate preferably resides on a medical device, most preferably a medical implant. The medical device preferably is selected from the group consisting of tissue engineering constructs, orthopedic implants, dental implants, and ventricular assist devices.

The substrate comprises a biocompatible material. The biocompatible material preferably is selected from the group consisting of silicon, metals, metal alloys, and ceramics. A preferred metal comprises titanium, and may be titanium mental or an alloy thereof. A preferred ceramic comprises hydroxyapatite.

The passivating coating comprises keratin and, in a preferred embodiment, one or more bioactive factors selected from the group consisting of bone morphogenetic protein (BMP) and transforming growth factor beta (TGF-β). The keratin is derived from a material selected from the group consisting of hair, fur, feathers, horns, hooves, beaks, or feet. The keratin preferably is derived from hair, more preferably from human hair, which may be referred to as "human hair keratin." The keratin also preferably comprises reduced keratin.

The passivating coating preferably comprises a bonding region and a bioactive region. The bonding region comprises a material adapted to bond with both a surface of the implant and with reactive pendant groups on the keratin. Preferably, the bonding region comprises at least one silane compound, more preferably an organosilane.

The organosilane preferably comprises a silane component and an organic component. The bonding region comprises the silane component bonded with a surface of the medical device and the bioactive region comprises the keratin bonded to the organic component of the organosilane. Suitable organic components for the organosilane are adapted to react with reactive pendant groups on the keratin. The organosilanes preferably comprise an organic component comprising a moiety selected from the group consisting of epoxy groups, alkoxy groups, vinyl groups, amine groups, isocyanate groups, and carboxyl groups. Preferred organic components comprise a moiety selected from the group consisting of epoxy groups, alkoxy groups, vinyl groups, and amine groups. More preferred organic components are selected from the group consisting of epoxy groups, acrylate groups, alkoxy groups, vinyl groups, and alkylamine groups. Most preferred organic moieties are selected from the group consisting of vinyl groups and epoxy groups. The organosilane preferably comprises substituents selected from the group consisting of from about 1 to 3 halogens and from about 1 to 3 alkoxy groups. A preferred halogen is chlorine.

Also provided is a method of coating a substrate with a passivating substrate. The method comprises: bonding a coupling material to said substrate, producing a bonding region; and, bonding keratin to said bonding region. The method further preferably comprises oxidizing a surface of said substrate before bonding a coupling material to the substrate.

In a preferred embodiment the substrate is cleaned before bonding the coupling material to the substrate. Most

preferably, the cleaning comprises sonication in anhydrous solvent and sonicaton in water. Suitable anyhydrous solvents include, but are not necessarily limited to methanol, ethanol, isopropyl alcohol, dimethylsulfoxide, acetone, or tetrahydrofuran. A preferred anhydrous solvent is dichloromethane. The water preferably is deionized water.

Bonding keratin to said bonding region comprises dissolving keratin in an appropriate solvent and adding an anhydrous solvent to produce a keratin mixture, exposing said bonding region to said keratin fixture; and curing under conditions effective to produce said bioactive region.

For reduced/reduced keratins, an appropriate solvent is water. For oxidized/reduced keratins, an appropriate solvent comprises an aqueous solution comprising a base. Suitable bases include, but are not necessarily limited to ammonium hydroxide, sodium hydroxide, and/or potassium hydroxide. A preferred base is ammonium hydroxide.

Suitable anhydrous solvents include, but are not necessarily limited to methanol, ethanol, isopropyl alcohol, dimethylsulfoxide, acetone, or tetrahydrofuran. A preferred anhydrous solvent is dimethylsulfoxide.

The method further comprises mixing the keratin mixture with a reagent selected from the group consisting of a catalyst and an initiator. When a vinyl-functional silane coupling agent is used, a suitable initiator or catalyst generates free radicals. A preferred free radical initiator is selected from the group consisting of anthraquinone-2-sulfonic acid or anthraquinone-2-sulfonic acid sodium salt monohydrate (Aldrich; Milwaukee, WI).

Curing comprises exposing the substrate that is exposed to the keratin mixture to an effective energy source for a period of time sufficient to cure the coating. The curing preferably occurs in the presence of said catalyst or said initiator. The period of time for curing typically is from about 1 to about 24 hours, preferably about 24 hours.

## Brief Description of the Figures

Figure 1 as a graph showing the water contact angle of keratin-coated titanium disks, which demonstrates that disks subjected to the process of the present application, silane coating followed by grafting of keratins, had the lowest resulting contact angle.

Figure 2 is a graph showing the water contact angle of keratin-coated substrates demonstrating that both titanium and glass were effectively coated using the dipping process.

Figure 3 is a chart comparing XPS spectra between an untreated titanium disk and a) a silane-coated disk (Si2p spectra) and b) a disk treated with silane, catalyst and keratin (N1s spectra).

Figure 4 is a graph showing Si and N XPS atomic concentrations of keratin-coated titanium disks.

Figure 5 is the estimated thickness of coatings on titanium disks, where the numbers in parenthesis indicate the thickness of the films on the silane coating.

Figure 6 is a micrograph showing that 2T3 cells adhere to and differentiate on keratin-coated titanium disks.

## Detailed Description

Keratins are one of the few, if not the only family of human proteins that can readily be donated without injury or trauma. This unique family of structural proteins can be found in numerous vertebrate tissues, most notably in hair and fur. Hundreds of tons of human hair are disposed of every day. As a family with more than 100 homologues, the biocompatibility of keratins is predetermined by their structural variation. No other human protein offers this magnitude of structural and compositional diversity, and therefore, tolerance by the immune system.

The present application selectively extracts keratins from human hair fibers and uses the keratins in a dipping process which produces a molecular coating on the surfaces of medical device materials, such as metals and ceramic. The method uses a coupling agent adapted to bond with both a surface of the implant and with reactive pendant groups on the keratin. Preferably, the bonding region comprises at least one silane compound, more preferably an organosilane. The organosilane preferably comprises a silane component and an organic component, wherein the bonding region comprises the silane component bonded with a surface of the medical device and the organic component bonded with reactive moieties on the keratin. In a preferred embodiment, the silane component of the organosilane is covalently bonded with the surface of the medial device and the organic component of the organosilane is covalently bonded with reactive moieties on the keratin.

Characterization of these coatings by water contact angle and X-ray photoelectron spectroscopy (XPS) revealed highly altered surface properties. The angstroms-thin keratin coating was robust, and did not interfere with the normal topology of grit blasted titanium disks. These surface properties were shown to be conducive to the attachment, growth, and differentiation of 2T3 mouse osteoblasts on keratin coated titanium disks in culture.

The coating method is generally applicable to medical device materials, which typically comprise metal and ceramic surfaces. The coating method has the potential of producing a more biocompatible interface and ultimately, biologically active surfaces that promote host acceptance and/or accelerate tissue ongrowth.

**Keratins**

Human hair is composed of a tough, tubular outer layer made up of flattened cells arranged in a scaly, overlapping profile known as the cuticle. The inner bulk of the hair is known as the cortex and is constructed from elongated cells that are densely packed with fibrous keratins. The fibrous keratins are arranged in bundles, referred to as macrofibrils, that contain microfibrillar coiled-coils of α-helical keratins. The intermediate filaments (IFs) of this structure are bound together with an amorphous keratin matrix material. The matrix and IFs vary in function and composition. The matrix is the "glue" that holds the IFs together. This glue is high in sulfur content and is made up of low molecular weight keratins (LMWKs), typically less than 10-15 kDa. The IFs are composed of keratins of relatively low sulfur content but high molecular weight (high molecular weight keratins or HMWKs), generally 50-85 kDa.

One distinguishing characteristic of keratins is that they are afforded their structural integrity in large part by the presence of disulfide crosslinks which form a three dimensional network of polypeptide chains. This network structure renders keratins insoluble. Keratins can, however, be made soluble by destroying this three-dimensional structure via disulfide bond scission. Disulfide bond scission can be done oxidatively, reductively, or by a combination of the two.

Disulfide bond scission and selective extraction under controlled conditions results in the isolation of LMWKs from HMWKs. This process serves to remove the cortical proteins without disrupting the cuticle. The inner structure of the hair fiber is removed without substantial damage to the cuticle. The HMWKs from this extraction process give rise to two broad protein bands by gel electrophoresis at molecular weight of 66 kDa and 43 kDa. These keratins are useful as structural biomaterials, including coatings for ceramic and metal surfaces.

Growth factors, such as bone morphogenetic protein (BMP) and transforming growth factor beta (TGF-β), are present in the hair follicle, are deliverable by the coatings, and provide the coatings with osteoinductive and osteoconductive properties.

Keratin comprises amino acids, which generally have the formula:

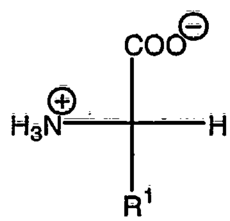

Table 1 summarizes the amino acid residues found in human hair, for example, and shows the "$R^1$" groups associated with each residue.

| Table 1. Ranked average amounts of amino acid in human hair | | | | | |
|---|---|---|---|---|---|
| Amino Acid | $R^1$ Group | Nature | pKa | Isoelectric Point (pH) | Percent Composition in Hair |
| Cysteine | $H\text{-}S\text{-}CH_2\text{-}$ | Nonpolar | 8.4 | 5.02 | 17.3 |
| Glutamic Acid | 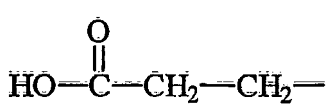 | Polar | 4.5 | 3.22 | 13.9 |
| Arginine | 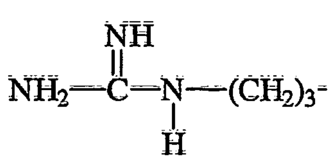 | Polar | 12.5 | 11.15 | 9.85 |
| Serine | $HO\text{-}CH_2\text{-}$ | Polar | None | 5.68 | 9 |

(continued)

| Table 1. Ranked average amounts of amino acid in human hair | | | | | |
|---|---|---|---|---|---|
| Amino Acid | $R^1$ Group | Nature | pKa | Isoelectric Point (pH) | Percent Composition in Hair |
| Threonine | 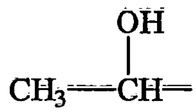 | Polar | None | 5.64 | 7.75 |
| Leucine | 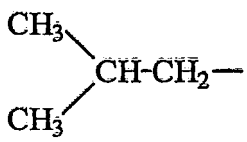 | Hydro phobic | None | 5.98 | 7.35 |
| Proline | 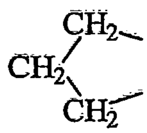 | Hydro phobic | None | 6.3 | 6.95 |
| Aspartic Acid | 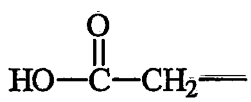 | Polar | 4.5 | 2.77 | 5.8 |
| Valine | 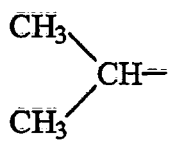 | Hydro phobic | None | 5.96 | 5.7 |
| Isoleucine | 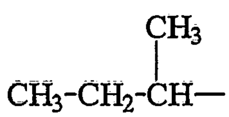 | Hydro phobic | None | 5.94 | 4.75 |
| Glycine | H- | Nonpolar | None | 5.65 | 4.15 |
| Phenylalanine | 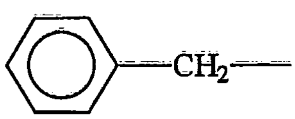 | Hydro phobic | None | 5.48 | 3 |
| Alanine | $CH_3$- | Hydrophobic | None | 6 | 2.8 |
| Tyrosine | 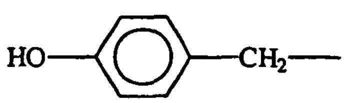 | Hydro phobic | None | 5.66 | 2.6 |
| Lysine | $NH_2-(CH_2)_4-$ | Polar | 10.4 | 9.59 | 2.5 |
| Histidine | 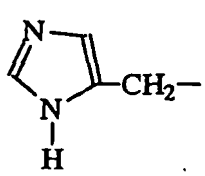 | Aromatic | 6.2 | 7.47 | 0.9 |
| Methionine | $CH_3-S-CH_2-CH_2-$ | Hydro phobic | None | 5.74 | 0.85 |

(continued)

| Table 1. Ranked average amounts of amino acid in human hair | | | | | |
|---|---|---|---|---|---|
| Amino Acid | $R^1$ Group | Nature | pKa | Isoelectric Point (pH) | Percent Composition in Hair |
| Tryptophan | 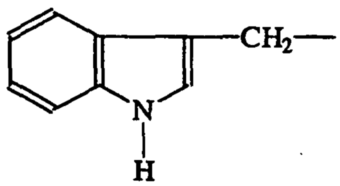 | Hydro phobic | None | 5.89 | 0.85 |

The most abundant amino acid in human hair is cysteine, which is found in the form of disulfide-bridged cystine groups. As discussed above, this group can be converted to other sulfur containing moieties, most notably thiol. Thiols theoretically can be reacted with reactive ends of the coupling material using a number of chemical techniques, such as those described in S. Patai (Ed.), the Chemistry of the Thiol Group, Parts 1 and 2, John Wiley & Sons, New York, NY (1974). Other reaction scenarios, such as those directed toward polymer synthesis, also are useful to utilize thiols to form an assortment of desirable crosslinks, including those described in Rempp, P. and Merrill, E. W., Polymer Synthesis, Huethig & Wepf Verlag Basel, Heidelberg, Germany (1986); Young, R. J. and Lovell, P. A., Introduction to Polymers, Chapman & Hall, London (1991); Odian, G., Principles of Polymerization, John Wiley & Sons, New York, NY (1991).

In addition to cysteine, the following amino acids have pendant groups comprising nitrogen or oxygen which may be useful as reactive pendant groups; arginine, serine, glutamic acid, threonine, aspartic acid, lysine, asparagine, glutamine, tyrosine, tryptophan, and histidine. Where the protein is α-keratin, preferred amino acid residues comprising reactive pendant groups for crosslinking are cysteine, arginine, serine, and glutamic acid, most preferably cysteine and arginine.

**Disulfide bond scission and keratin extraction**

The keratins may be processed and/or isolated in a number of ways. Preferably, the processing is sufficient to render the resulting proteins water soluble. Suitable processing techniques include known oxidation techniques, reductive techniques, and/or combinations thereof, as long as the processing renders the proteins water soluble without significant hydrolysis of peptide bonds.

A number of reductive chemistries are known for disulfide bond scission in keratins: See Wardell, J. L., "Preparation of Thiols" in The Chemistry of the Thiol Group, Patai, S. (Editor), pp. 163-353, John Wiley & Sons, New York, NY (1974). HMWK's may be extracted from hair using at least two reductive extractions; as described in Crewther, W. G., Fraser, R. D. B., Lennox, F. G., and Lindley, H., "The Chemistry of Keratins" in Advances in Protein Chemistry, Anfinsen, C. B., Jr., Anson, M. L., Edsall, J. T., and Richards, F. M. (Editors), Academic Press, New York, pp. 191-346 (1965).

The following methods are suitable for processing keratins for use in producing the coatings:

**-Oxidation/ Reduction**

In a preferred embodiment, which uses keratins as a source material (e.g. human hair), the hair is oxidized by a suitable oxidizing agent. Suitable oxidizing agents include, but are not necessarily limited to hydrogen peroxide, peracetic acid, percarbonates, persulfates, chlorine dioxide, sodium and calcium peroxides, perborates, and hypochlorite. A most preferred oxidizing agent is hydrogen peroxide. The oxidants are used at a concentration of up to about 35%, preferably at from about 0.1% to about 10%. The oxidation preferably occurs at reflux temperatures.

In a preferred embodiment, the hair is treated with hydrogen peroxide ($H_2O_2$), at from about 0.1% to about 10%, most preferably 1%, in order to disrupt the cuticle and swell the keratin source material. This process also converts some fraction of the cystine residues into sulfonic acid groups. The amount of oxidation may be controlled by varying the time of oxidation, preferably from about 0 hours to about 4 hours, while retaining the other conditions of the oxidation reaction constant. These conditions include concentration and type of oxidant, temperature, and ratio of extracting media to keratin source material. After the reaction is complete, the oxidized hair is filtered and rinsed, preferably with deionized water. The filtrate is discarded and the hair allowed to dry.

Where other conditions of oxidation are maintained constant, the conversion rate of cystine to sulfonic acid residues is roughly proportional to the amount of time used for the oxidation. Residual cystines in the resulting oxidized keratin solids are converted to other sulfur-containing moieties using reductive techniques. Preferably, the disulfide-bridged cystine group is converted to a thiol group, which has utility of it's own, or can be modified using a variety of chemical techniques.

The oxidized hair preferably is treated with a reducing agent. Suitable reducing agents include, but are not necessarily limited to thioglycolic acid and salts thereof, mercaptoethanol, dithiothreitol, thioglycerol, thiolactic acid, glutathione, cysteine, sodium sulfide, and sodium hydrosulfide. Preferred reducing agents are thioglycolic acid and mercaptoethanol, most preferably thioglycolic acid.

In order to treat the oxidized hair with the reducing agent, the previously oxidized hair is suspended in the reducing agent typically at a concentration of up to about 10N, preferably from about 0.1N and 1N; at a pH greater than about 7, preferably equal to or greater than 9, most preferably 9; a temperature of from about 25 to about 80˚C, preferably about 60 ˚C, preferably for a time period of from about 1 to about 72, most preferably about 24 hours. The reaction occurs under an inert atmosphere, preferably nitrogen. The liquid fraction is separated from any remaining solids using known means, including but not necessarily limited to filtration, or cannulation and/or centrifugation, preferably under inert atmosphere. A preferred method of separation is filtration. Once the solids are removed, the soluble keratin proteins are isolated from the solution by addition of a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The resulting keratin proteins are dried using known techniques, preferably overnight under vacuum at room temperature. This process results in the keratins having both sulfonic acid groups and thiol groups.

In a most preferred reaction, clean, dry human hair is treated with hydrogen peroxide with heating, preferably at reflux, for a period of time effective to achieve scission of disulfide bonds, typically about 180 minutes. The hair is separated from the liquid, preferably by filtration, and the liquid is discarded. The hair was rinsed with water, preferably in copious amounts, and allowed to air dry. The dried, oxidized hair is then treated with a reducing agent, preferably 1M thioglycolic acid at pH 9 (adjusted with ammonium hydroxide), and the mixture is heated, preferably to about 60 ˚C, under inert gas, preferably under a nitrogen atmosphere, for a period of time, preferably 24 hours. After reductive extraction, the solids are separated from the liquid, preferably by centrifugation. The filtered liquid is added, preferably dropwise, to an excess of ethanol, preferably about an 8-fold volume excess of ethanol, thereby forming a keratin precipitate. The precipitated keratins are isolated by filtration and dried under vacuum. The keratin is ground into a fine powder using a mortar and pestle.

## Reductive/reductive extraction

In another embodiment, a first reductive extraction is performed by treating the hair with a first reducing agent under first conditions effective to selectively extract matrix keratins, producing a first solution comprising soluble reduced matrix keratins (LMWK's) and remaining hair solids (HMWK's). Although it may be possible to subject the LMWK's to the techniques described herein to produce coatings, preferred proteins for use in the techniques herein are HMWK's, which preferably are isolated during a second extraction. The remaining hair solids and the first solution are separated, and the remaining hair solids are exposed to a second extraction solution under second conditions effective to solubilize α-keratins, producing a second solution comprising soluble reduced α-keratins (HMWK's) and solid cuticle.

Suitable reducing agents again include, but are not necessarily limited to thioglycolic acid and salts thereof, mercaptoethanol, dithlothreitol, thioglycerol, thiolactic acid, glutathione, cystine, sodium sulfide, and sodium hydrosulfide. Preferred reducing agents are thioglycolic acid and mercaptoethanol, most preferably thioglycolic acid.

In order to selectively reduce and extract the desired proteins, the hair (or other protein source) is suspended in a reducing agent at a concentration of from about 0.1M to about 10M, preferably about 1.0M. Gentle swelling of hair fibers is achieved at a pH of about 9 or more, preferably at a pH of from about 9 to about 10.5. Hence, the initial reduction takes place at a temperature of from about 20 to about 100 ˚C, preferably at about 25 ˚C. The time period required to accomplish the first reduction is from about 8 to about 36 hours, most preferably about 24 hours. The reaction occurs under an inert atmosphere, preferably nitrogen. The liquid fraction is separated from remaining solids using known means, including but not necessarily limited to filtration, cannulation, and/or centrifugation, preferably under inert atmosphere. A preferred method of separation is filtration.

A second extraction is performed using a suitable swelling agent, preferably urea, and/or a base such as ammonium hydroxide, sodium hydroxide, or potassium hydroxide. A most preferred swelling agent for this second extraction is concentrated urea. The second extraction effectively removes the fibrous α-keratins from inside the cuticle. The second extraction occurs at from about 1M to about 10M urea, preferably about 7M urea, for a period of at least about 1 hour, preferably from about 1 to about 72 hours, more preferably 20 hours or more, most preferably about 24

hours. The second extraction occurs at room temperature, but may take place at temperatures of from about 20 °C to about 100 °C, preferably about 25 °C. The liquid fraction is separated from the empty, intact cuticle, using known means. Suitable means include but are not necessarily limited to filtration, cannulation and/or centrifugation, preferably under inert atmosphere. A preferred method of separation is filtration.

Once the cuticle is removed, the water soluble keratin proteins may be retained in solution for further use, or they may be isolated from the solution by addition to a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The precipitated proteins are dried using known techniques, preferably overnight under vacuum at room temperature. The extracted water soluble keratin proteins (herein sometimes collectively referred to as "water soluble proteins") comprise thiols or thiol groups.

## Coating Process

### -Cleaning the surface

Before coating the substrate, the surface is cleaned using any suitable procedure. An example of a suitable procedure is sequential sonication in an anhydrous solvent and water. Suitable anyhydrous solvents include, but are not necessarily limited to methanol, ethanol, isopropyl alcohol, dimethylsulfoxide, acetone, or tetrahydrofuran. A preferred anhydrous solvent is dichloromethane. The water preferably is deionized water, followed by drying, preferably in air.

### Forming the Bonding Region

After cleaning, the implant is immersed in a solution of the coupling material, preferably an organosilane. The use of silane compounds in surface coatings is a well-known industrial process. Arkels B. "Tailoring surfaces with silanes." Chemtech 1977;7:766-778. Not only can organosilanes passivate and change the chemistry of a surface, they can be used as coupling agents to facilitate the bonding of yet another compound to the outside surface of a substrate.

There are many different types of commercially available organosilanes with a variety of chemical functionalities. The organosilanes preferably comprise an organic component comprising a moiety selected from the group consisting of epoxy groups, alkoxy groups, vinyl groups, amine groups, isocyanate groups, and carboxyl groups. Preferred organic components comprise a moiety selected from the group consisting of epoxy groups, alkoxy groups, vinyl groups, and amine groups. More preferred organic components are selected from the group consisting of epoxy groups, methacrylate groups, alkoxy groups, vinyl groups, and alkylamine groups. Most preferred organic moieties are selected from the group consisting of vinyl groups and epoxy groups.

The organosilane preferably comprises substituents selected from the group consisting of from about 1 to 3 halogens and from about 1 to 3 alkoxy groups. A preferred halogen is chlorine.

A preferred organosilane, particularly where the surface is titanium, is vinylmethyldichlorosilane (Gelest Inc., Tullytown, PA). The organosilane is in an organic solvent. The organic solvent may be substantially any organic solvent that is effective to maintain the organosilane in solution without negatively impacting the bonding process. The organic solvent preferably comprises elements other than oxygen and/or nitrogen. Examples of suitable organic solvents include, but are not necessarily limited to alkanes, alkylene chlorides, chloroform, xylenes, and combinations thereof. A preferred alkane is hexane and a preferred alkylene chloride is methylene chloride. A most preferred organic solvent is hexane. The percentage of the organosilane in the organic solvent may vary from about 1 to about 10 weight percent, preferably about 1 weight percent.

The substrate is maintained in the solution for a time sufficient to form the coating, suitably from about 1 to about 30 minutes, typically about 10 minutes. The substrate then is removed from solution, rinsed with copious amounts of fresh organic solvent, and allowed to air dry.

### -Forming the bioactive region

To form the bioactive region, keratin powder obtained as described above is dissolved in an appropriate solvent. For reduced/reduced keratins, an appropriate solvent is water. For oxidized/reduced keratins, an appropriate solvent comprises an aqueous solution comprising a base. Suitable bases include, but are not necessarily limited to ammonium hydroxide, sodium hydroxide, and/or potassium hydroxide. A preferred base is ammonium hydroxide. In a preferred embodiment, about 15 grams of the keratin powder is dissolved in about 30 mL of 3N ammonium hydroxide.

An anhydrous solvent is added. Suitable anhydrous solvents include, but are not necessarily limited to methanol, ethanol, isopropyl alcohol, dimethylsulfoxide, acetone, or tetrahydrofuran. A preferred anhydrous solvent is dimethylsulfoxide. At the foregoing weight and volume of keratin and base, about 270 mL of dimethylsulfoxide is added. The mixture is stirred with mild heating until the keratin is dissolved.

A suitable catalyst or initiator preferably is used. For example, wherein a vinyl-functional silane coupling agent is used, a suitable initiator or catalyst is used to generate free radicals. An example of such an initiator is a UV activated photoinitiator, such as anthraquinone-2-sulfonic acid or anthraquinone-2-sulfonic acid sodium salt monohydrate (Aldrich; Milwaukee, WI). At the foregoing weights and volumes, about 1.5 grams of anthraquinone-2-sulfonic acid sodium salt monohydrate is added. The resulting solution is poured over the silane-coated implant such that it is completely submerged. The coating on the submerged substrate then is cured using an effective energy source, such as a heat lamp, an autoclave, a microwave, or a UV lamp. A preferred energy source is a UV lamp ($\lambda$=365 nm, 1.05 amps). Curing is continued for a sufficient period of time to cure the coating, typically from about 1 to about 24 hours, preferably for about 24 hours. After curing, the substrate is removed from the solution, rinsed, preferably with copious amounts of deionized water, and allowed to dry, preferably by air.

Persons of ordinary skill in the art will recognize that the foregoing parameters, such as weights, volumes, and times, may be varied depending upon the surface area of the implant, the particular bonding material used, and other factors.

The application will be better understood with reference to the following examples, which are illustrative only, and should not be construed as limiting the claims to a particular embodiment. The following materials and methods were used in the examples described below:

## Materials and Methods

*Preparation of Keratin*

In a typical reaction, 500 g of clean, dry human hair was placed in a 12 L round bottom flask. 8,350 mL of 1 weight/volume percent of hydrogen peroxide was added and the reaction heated to reflux for 180 minutes. The hair was separated from the liquid by filtration and the liquid discarded. The hair was rinsed with copious amounts of water and allowed to air dry. 100 g of the dried, oxidized hair was placed in a 2000 mL round bottom flask, 1000 mL of 1M thioglycolic acid at pH 9 (adjusted with ammonium hydroxide) was added and the mixture was heated to 60˚C under a nitrogen atmosphere for 24 hours. After reductive extraction, the solids were separated from the liquid by centrifugation. The filtered liquid was added dropwise to an 8-fold volume excess of ethanol thereby forming a keratin precipitate. The precipitated keratins were isolated by filtration and dried under vacuum. The keratin was ground into a fine powder using a mortar and pestle.

*Titanium disks*

Grit blasted titanium test disks with highly controlled and thoroughly characterized surfaces to be used for cellular response were prepared. The material chosen was a medical grade, commercially pure titanium (C.P. Ti). The materials were purchased as large sheets (approximately 5 ft. x 4 ft. x 0.050 in., to ensure metallurgical homogeneity for all sample sets) from Timet (O'Fallon, MO, USA).

1 ft. x 1 ft. sheets were cut from the original large single sheet. In order to ensure a consistent surface finish for each disk, surface preparation was performed on these sheets. Each 1 ft. x 1 ft. sheet was subjected to the aluminum oxide grit blasting process with compressed air using a #20 grit aluminum oxide ($Al_2O_3$) powder for the rough surface finish; grit blasting was performed by Southwest Research Institute (San Antonio, TX, USA).

Quality control of the surface finishing operations was performed and gauged by the consistency of surface roughness measurements. The surface roughness was measured using a diamond stylus contact profilometer at 10 different locations on each 1 ft. x 1 ft. plate. The roughness measurements were taken in all directions. The average $R_a$ was 9.56 $\mu$m with a standard deviation of 1.63 $\mu$m.

Disks with 14.75 mm diameter and 0.80mm thickness were prepared for the cell assays. The disks were washed 3 times in a detergent solution and rinsed 2 times with DI water in between each wash. After the final rinse, ethanol was poured over the disks to remove excess water. The disks were air-dried.

*Costing of Titanium Disks*

In a typical procedure, titanium disks were cleaned by sequential sonication in dichloromethane and deionized water followed by drying in air. Disks were immersed in a 1 weight percent solution of vinylmethyldichlorosilane (Gelest Inc., Tullytown, PA) in hexanes for 10 minutes. Each disk was individually removed from solution, rinsed with copious amounts of fresh hexanes, and allowed to air dry.

In a typical keratin coating step, 15 grams of the keratin powder was dissolved in 30 mL of 3N ammonium

hydroxide. 270 mL of dimethylsulfoxide was added and the mixture stirred with mild heating until the keratin dissolved. 1.5 grams of anthraquinone-2-sulfonic acid sodium salt monohydrate (Aldrich; Milwaukee, WI) was added and the solution poured over the silane-coated titanium disk samples such that they were completely submerged in a shallow glass dish. The glass dish was placed under a UV lamp ($\lambda$=365 nm, 1.05 amps) for 24 hours. After UV exposure, each disk was removed from solution, rinsed with copious amounts of deionized water, and allowed to air dry.

*Water Contract Angle*

Contact angles were measured on an optical comparator by placing a 100-$\mu$L drop of ultrapure water on the horizontal substrate. Interfacial energies were allowed to equilibrate for 30 seconds prior to obtaining a measurement.

*X-ray Photoelectron Spectroscopy (XPS)*

XPS data was obtained using a Physical Electronics PHI5700 ESCA system with an Al monochromatic source (Al K$\alpha$ radiation at 1486.6 eV). The base pressure in the XPS ultrahigh vacuum chamber was 2 X $10^{-10}$ torr during the analysis. High resolution scans were obtained with a step size of 0.1 eV and pass energy of 11.75 eV. The presence of the silane and keratin coatings was monitored by tracking the atomic concentration of elements that are only present in each of these coatings. Silicon was used for the silane coating and nitrogen for the keratin. Although sulfur is also present in keratin and detected by XPS, it was not used in this work as the signal-to-noise ratio for sulfur was close to 1.5; therefore, the atomic concentrations calculated for the sulfur signal were in the same order as the error bars.

XPS was also used to estimate the film thickness. If the signal of a clean substrate is known, then the XPS signal from the substrate with a film on it can be calculated using the following standard attenuation equation

$$N = N_o \cdot \exp\left[-t/\left(\lambda \cdot \sin\theta\right)\right]$$

where $N$ is the XPS signal (area under the peak) of the substrate with a film on it, $N_o$ is the XPS signal of the clean substrate, $t$ is the thickness of the film, $\lambda$ is the mean-free path of photoelectrons in the film (assumed to be 20 Å in this work), and $\theta$ is the angle between the XPS detector and the surface, equal to 45 degrees in this case. C. S. Fadley, Progress in Surface Science 1984;16: 275-388.

*Osteoblast Cell Culture Assay*

Alpha-minimal essential medium ($\alpha$-MEM) was purchased from Gibco BRL (Grand Island, NY, USA) and fetal bovine serum (FBS) was from Summit Biotechnology (Fort Collins, CO, USA). Formalin was obtained from Electron Microscopy Sciences (Fort Washington, PA, USA). All other reagents were purchased from Sigma Chemical Co. (St. Louis, MO, USA).

Driven by the bone morphogenetic protein 2 (BMP-2) promoter, the 2T3 cell line has been isolated and cloned from a transgenic mouse. Ghosh-Choudhury N, Windle JJ, Koop BA, Harris MA, Guerrero, DL, Wozney JM, Mundy GR, and Harris SE. "Immortalized murine osteoblasts derived from BMP2-T-anigen expressing transgenic mice." Endocrinology 1996;137:331-339.

These cells undergo bone matrix formation *in vitro.* 2T3 cells were plated onto each titanium disk in a 12-well plate, one plate per group. The cells were cultured with $\alpha$-MEM supplemented with 10% FBS until they reached 90% confluence, approximately day 5. The medium was changed every other day. Once the cells reached confluence, the medium was changed to $\alpha$-MEM containing 5% FBS, 100 $\mu$g/ml ascorbic acid (AsA) and 5 mM $\beta$-glycerophosphate ($\beta$-GP) for the treatment group or to 5% FBS $\alpha$-MEM without AsA and $\beta$GP for the control group. The medium was changed every three days and fresh reagents were added up to day 14, which was the end of the treatment period. Cells were cultured at 37˚C, 5% $CO_2$.

*Von Kossa Staining*

The von Kossa cell staining was modified from a previously published procedure. Beresford JN, Graves SE, and Smoothy CA. "Formation of mineralized nodules by bone derived cells in vitro: a model of bone formation?." Am. J. Med. Genet. 1993;45:163-78.

To fix the 2T3 cells onto the titanium disks, the cells were first washed with PBS and fixed in 10% phosphate buffered formalin for 10 min. The formalin solution was aspirated and the cells were rinsed with deionized water ($dH_2O$).

Fresh 2% silver nitrate ($AgNO_3$) was added to the cells and the titanium disks with cells were then put under sunlight for 10 minutes. After 10 minutes, the $AgNO_3$ was removed and the 2T3 cells were rinsed under a steady stream of $dH_2O$. A 5% sodium thiosulfate ($Na_2S_2O_3$) solution was added to the cells for 3 minutes. The $Na_2S_2O_3$ was removed and the cells were rinsed again with $dH_2O$. The following ethanol (EtOH) series was applied to the cells: 95% EtOH for 30 sec., repeat once more followed by two 100% EtOH applications for 30 seconds each. The cells were then allowed to air dry.

*Scanning electron microscopy*

To obtain images of the surface morphology, the titanium disks with cells were gold/palladium (Au/Pd) coated for 10 seconds in a sputter coater. Images of the titanium surface were obtained on an Array scanning electron microscope (SEM) (Amray, Bedford, MA, USA) at 15 kV. Images were acquired at three magnifications: 100X, 500X, and 1, 000X.

*Statistical Methods*

Where indicated, differences between sample groups were determined using a two-tailed Student's t-Test with unequal variance. Statistical significance was established at *p* values of less than 0.05.

**Example 1**

In the present study, a vinyl-functional silane coupling agent was used to graft keratins onto coated glass and titanium substrates using free radical addition chemistry. A UV activated photoinitiator, anthraquinone-2-sulfonic acid, was used to generate free radicals, although thermal initiation with another catalyst could also be used. In the first step of this process, excellent coating of the substrates was achieved by dipping into a hexane solution of the silane. As shown in Figure 1, the resulting increase in water contact angle for titanium disks averaged 38 degrees. The treatment groups included various combinations of keratin coatings with and without the silane coupling, both in the presence and absence of the photoinitiator (designated as "cat." in Figure 1). This was done to ascertain the effectiveness of the two step coating approach. Although all of the sample groups resulted in a contact angle different than that of the control titanium group (statistical significance of $p \leq 0.05$), the full treatment of silane coating followed by covalent grafting of keratin resulted in the most impressive reduction in contact angle compared to the untreated control. The fully treated titanium disk samples had an average contact angle of 7.6 degrees. The results for glass substrates were similar with a reduction in contact angle between the untreated control and the fully treated sample of 16.5 degrees. The keratin treated glass had an average contact angle of 9.0 degrees. These data are shown in Figure 2. Once it was determined that both titanium disks and glass microscope slides could be coated, only the titanium disks were used in further evaluations.

As mentioned previously, the successful coatings were achieved by dipping the titanium disks into a hexanes solution of the silane. The XPS Si2p signals from the untreated titanium disk and a silane-coated titanium disk are shown in Figure 3a. The Si signal from the coated disk corresponded to an atomic concentration of ~20%, which compared to <3% for the untreated disk clearly indicates the presence of the silane coating. The small amount of Si on the untreated disks was likely due to surface contamination. The addition of keratin resulted in an increase of nitrogen atomic concentration from ~3 % (background level on an untreated disk) to 8.5%. Figure 3b shows the typical N1s XPS signal from an untreated titanium disk and that of the sample coated with silane, the catalyst and keratin. The atomic concentrations of Si and N measured on the different treatment groups are shown in Figure 4. The concentration of these two elements correlated very nicely with the presence of the organosilane and the keratin.

The addition of the catalyst and keratin on the silane coating resulted in a decrease in the atomic concentration of Si, which results from the attenuation of the silicon signal by the different additional coatings. The titanium XPS signal was used in the attenuation equation described above to calculate the overall film thickness. The results are shown in Figure 5. The variations in the thickness of the films with silanes + "cat" or keratin are probably due to variations in the thickness of the silane film itself. Nevertheless, it is clear from the cat + keratin film and the keratin-only film that the keratin film is more robust in the presence of the silane coating.

In order to exclude variations in the silane film and to more accurately determine the thickness of the keratin coating, the attenuation equation was used, treating the silane as the substrate and using the silicon signal from silage-coated disks to estimate the thickness of the keratin coatings on top of the silane film. The Si2p XPS signal from the disk coated only with silane was used as the clean substrate signal in this case. The results for several different samples are indicated in parentheses in Figure 5. The data indicates that the average thickness of the keratin coatings is ~ 8.5 Å.

Differentiation and mineralization of osteoblasts on keratin coated titanium disks were demonstrated by von Kossa analysis of the disk surfaces. Nodule formation is considered to be an important step toward mineralization *in vitro.*

Cells were cultured in $\alpha$-MEM containing 5% FBS in the presence (+) or absence (-) of 100 $\mu$g/ml ascorbic

acid and 5 mM β-glycerophosphate. Differentiation and mineralization of osteoblasts as demonstrated by von Kossa analysis (panels A and C) without (-) AsA and β-GP showed smaller and fewer nodules. After 2 weeks, 2T3 cells were observed to mineralize larger nodules on the implant surface in the presence (+) of 100 μg/ml AsA and 5mM β-GP (panels B and D). Images A and B were acquired at 100X magnification (scale bar = 100μm) and C and D at 1,000X magnification (scale bar = 10μm).

The mineralization of nodules *in vitro* appears to resemble the *in vivo* produced woven bone. After 2 weeks, 2T3 cells were observed to mineralize in culture on the implant surface with or without AsA and β-GP. Without AsA and β-GP, von Kossa staining of the cells at 2 weeks showed smaller and fewer nodules (Figure 6A and C). In contrast, there were more and larger nodules on AsA and β-GP treated disks (Figure 6B and D). The ability of AsA and β-GP to induce nodule formation in 2T3 osteoblasts on collagen coated substrates has been previously reported. In this study, we find similar results for keratin coated substrates, thus indicating biocompatibility.

The two step dipping process for applying a keratin coating onto titanium substrates resulted in films that were hydrophilic and robust as evidenced by the water contact angle and XPS data, respectively. This process should result in a more biostable coating as the keratin is covalently bonded to the surface. The coating process resulted in very thin layers of both silane and keratin and would not be expected to interfere with the surface topology often employed by implant manufacturers to promote cell attachment to roughened titanium implant surfaces. The 2T3 osteoblast assay showed that the coating promoted attachment, growth, and differentiation. The ability of these cells to produce bony matrix is demonstrative of the keratin coatings osteoconductive properties. In future studies we intend to demonstrate that these keratin coatings are capable of accelerating the rate of bone ongrowth.

## Claims

**1.** A medical device comprising:

a substrate comprising a passivating coating comprising keratin, said passivating coating comprising a bonding region and a bioactive region;
said bonding region comprising at least one organosilane compound comprising a silane component bound to a surface of said substrate; and
said bioactive region comprising an organic component of said organosilane bound to a reactive pendant group on said keratin.

**2.** The medical device of claim 1 wherein said silane component of said organosilane compound is covalently bonded with a surface of the substrate.

**3.** The medical device of claim 2 wherein said bioactive region comprises reactive pendant groups on said keratin covalently bonded to said organic component of said organosilane.

**4.** The medical device of claim 1 wherein said passivating coating is effective to increase bone matrix formation exhibited by cultured 2T3 mouse osteoblast cells.

**5.** The medical device of any previous claim selected from the group consisting of tissue engineering constructs, orthopedic implants, medical implants, dental implants, and ventricular assist devices.

**6.** The medical device of any previous claim wherein said substrate comprises a biocompatible material.

**7.** The medical device of claim 6 wherein the biocompatible material is selected from the group consisting of silicon, metals, metal alloys, and ceramics.

**8.** The medical device of claim 6 wherein said biocompatible material is selected from the group consisting of titanium and hydroxyapatite.

**9.** The medical device of any previous claim wherein said passivating coating comprises keratin and one or more bioactive factors selected from the group consisting of bone morphogenetic protein (BMP) and transforming growth factor beta (TGF-β).

**10.** The medical device of any previous claim wherein said keratin is derived from a material selected from the group consisting of hair, fur, feathers, horns, hooves, beaks, and feet.

11. The medical device of claim 10 wherein said keratin is human hair keratin.

12. The medical device of any previous claim wherein said keratin comprises reduced keratin.

13. The medical device of any previous claim wherein said keratin comprises high molecular weight keratin (HMWK) having a molecular weight of from about 50 to about 85 kDa.

14. The medical device of any previous claim wherein said passivating coating is effective to accelerate bone matrix formation by cultured 2T3 mouse osteoblasts.

15. The medical device of any previous claim wherein said organic component of said organosilane comprises a moiety selected from the group consisting of epoxy groups, alkoxy groups, vinyl groups, amine groups, isocyanate groups, and carboxyl groups.

16. The medical device of claim 15 wherein said amine groups are alkylamine groups.

17. The medical device of any previous claim wherein said organosilane comprises substituents selected from the group consisting of from about 1 to 3 halogens and from about 1 to 3 alkoxy groups.

18. A method of coating a medical device with a passivating coating, said method comprising:

bonding a coupling agent to one or more surfaces of said medical device, producing a bonding region; and
bonding keratin to said bonding region.

19. The method of claim 18 wherein bonding keratin to said bonding region comprises:

dissolving keratin in a solvent; and
adding a second anhydrous solvent to produce a keratin mixture;
exposing said bonding region to said keratin mixture, producing a keratin coated bonding region; and
curing said keratin coated bonding region under conditions effective to produce said bioactive region.

20. The method of claim 19 further comprising cleaning said one or more surfaces before bonding said coupling agent to said one or more surfaces.

21. The method of claim 19 or 20 further comprising oxidizing said one or more surfaces before bonding said coupling agent to said one or more surfaces.

22. The method of claim 20 wherein said cleaning comprises sonicating said one or more surfaces in first anhydrous solvent and sonicating said one or more surfaces in water.

23. The method of claim 22 wherein said first anhydrous solvent is selected from the group consisting of methanol, ethanol, isopropyl alcohol, dimethylsulfoxide, acetone, tetrahydrofuran, and dichloromethane.

24. The method of claim 22 or 23 wherein said water is deionized water.

25. The method of any of the claims 19 to 24 wherein said second anhydrous solvent is selected from the group consisting of methanol, ethanol, isopropyl alcohol, dimethylsulfoxide, acetone, and tetrahydrofuran.

26. The method of any of claims 19 to 25 further comprising mixing the keratin mixture with an activation agent selected from the group consisting of a catalyst and an initiator.

27. The method of any of the claims 19 to 26 wherein said conditions comprises exposing said keratin coated bonding region to an energy source for a period of time effective to produce said bioactive region.

28. The method of claim 27 wherein said conditions comprise the presence of said activation agent.

29. The method of claim 27 or 28 wherein said period of time is from about 1 to about 24 hours.

30. The method of claim 29 wherein said period of time is about 24 hours.

**Patentansprüche**

1. Medizinische Einheit, umfassend:

   ein Substrat, umfassend eine passivierende Beschichtung, die Keratin umfasst, wobei die passivierende Beschichtung einen Bindungsbereich und einen bioaktiven Bereich umfasst;
   wobei der Bindungsbereich wenigstens eine Organosilanverbindung umfasst, die eine an eine Oberfläche des Substrats gebundene Silanverbindung umfasst; und
   wobei der bioaktive Bereich eine organische Komponente des Organosilans umfasst, der an eine reaktive Seitengruppe an dem Keratin gebunden ist.

2. Medizinische Einheit gemäß Anspruch 1, wobei die Silankomponente der Organosilanverbindung kovalent an eine Oberfläche des Substrats gebunden ist.

3. Medizinische Einheit gemäß Anspruch 2, wobei der bioaktive Bereich reaktive Seitengruppen an dem Keratin umfasst, die kovalent an die organische Komponente des Organosilans gebunden sind.

4. Medizinische Einheit gemäß Anspruch 1, wobei die passivierende Beschichtung zum Erhöhen der Knochenmatrixbildung, die von kultivierten 2T3-Maus-Osteoblastenzellen gezeigt wird, wirksam ist.

5. Medizinische Einheit gemäß einem der vorstehenden Ansprüche, ausgewählt aus der Gruppe bestehend aus Gewebe-Engineering-Konstrukten, orthopädischen Implantaten, medizinischen Implantaten, Dentalimplantaten und Einheiten zur ventrikulären Unterstützung.

6. Medizinische Einheit gemäß einem der vorstehenden Ansprüche, wobei das Substrate ein biokompatibles Material umfasst.

7. Medizinische Einheit gemäß Anspruch 6, wobei das biokompatible Material ausgewählt ist aus der Gruppe bestehend aus Silicium, Metallen, Metalllegierungen und Keramik.

8. Medizinische Einheit gemäß Anspruch 6, wobei das biokompatible Material ausgewählt ist aus der Gruppe bestehend aus Titan und Hydroxyapatit.

9. Medizinische Einheit gemäß einem der vorstehenden Ansprüche, wobei die passivierende Beschichtung Keratin und einen oder mehrere bioaktive Faktoren umfasst, die ausgewählt sind aus der Gruppe bestehend aus Knochenmorphogenetischem Protein (BMP) und dem transformierenden Wachstumsfaktor beta (TGF-β).

10. Medizinische Einheit gemäß einem der vorstehenden Ansprüche, wobei das Keratin aus einem Material erhalten ist, das ausgewählt ist aus der Gruppe bestehend aus Haar, Fell, Federn, Hörnern, Hufen, Schnäbeln und Füßen.

11. Medizinische Einheit gemäß Anspruch 10, wobei das Keratin menschliches Haarkeratin ist.

12. Medizinische Einheit gemäß einem der vorstehenden Ansprüche, wobei das Keratin reduziertes Keratin umfasst.

13. Medizinische Einheit gemäß einem der vorstehenden Ansprüche, wobei das Keratin Keratin mit hohem Molekulargewicht (HMWK) umfasst, das ein Molekulargewicht von etwa 50 bis etwa 85 kDa aufweist.

14. Medizinische Einheit gemäß einem der vorstehenden Ansprüche, wobei die passivierende Beschichtung zum Beschleunigen der Knochenmatrixbildung durch kultivierte 2T3-Maus-Osteoblasten wirksam ist.

15. Medizinische Einheit gemäß einem der vorstehenden Ansprüche, wobei die organische Komponente des Organosilans eine Einheit umfasst, die ausgewählt ist aus der Gruppe bestehend aus Epoxygruppen, Alkoxygruppen, Vinylgruppen, Amingruppen, Isocyanatgruppen und Carboxygruppen.

16. Medizinische Einheit gemäß Anspruch 15, wobei die Amingruppen Alkylamingruppen sind.

**17.** Medizinische Einheit gemäß einem der vorstehenden Ansprüche, wobei das Organosilan Substituenten umfasst, die ausgewählt sind aus der Gruppe bestehend aus etwa 1 bis 3 Halogenatomen und etwa 1 bis 3 Alkoxygruppen.

**18.** Verfahren zum Beschichten einer medizinischen Einheit mit einer passivierenden Beschichtung, wobei das Verfahren Folgendes umfasst:

Binden eines Kupplungsmittels an eine oder mehrere Oberflächen der medizinischen Einheit, wobei ein Bindungsbereich hergestellt wird; und
Binden von Keratin an den Bindungsbereich.

**19.** Verfahren gemäß Anspruch 18, wobei das Binden von Keratin an den Bindungsbereich Folgendes umfasst:

Lösen von Keratin in einem Lösungsmittel; und
Zugeben eines zweiten, wasserfreien Lösungsmittels zum Herstellen eines Keratingemischs;
Exponieren des Bindungsbereichs an das Keratingemisch, wobei ein keratinbeschichteter Bindungsbereich hergestellt wird; und
Aushärten des keratinbeschichteten Bindungsbereichs unter Bedingungen, die zum Herstellen des bioaktiven Bereichs wirksam sind.

**20.** Verfahren gemäß Anspruch 19, ferner umfassend das Reinigen der einen oder mehreren Oberflächen vor dem Binden des Kupplungsmittels an die eine oder mehreren Oberflächen.

**21.** Verfahren gemäß Anspruch 19 oder 20, ferner umfassend das Oxidieren der einen oder mehreren Oberflächen vor dem Binden des Kupplungsmittels an die eine oder mehreren Oberflächen.

**22.** Verfahren gemäß Anspruch 20, wobei das Reinigen Ultraschallbehandeln der einen oder mehreren Oberflächen in einem ersten, wasserfreien Lösungsmittel und Ultraschallbehandeln der einen oder mehreren Oberflächen in Wasser umfasst.

**23.** Verfahren gemäß Anspruch 22, wobei das erste, wasserfreie Lösungsmittel ausgewählt ist aus der Gruppe bestehend aus Methanol, Ethanol, Isopropylalkohol, Dimethylsulfoxid, Aceton, Tetrahydrofuran und Dichlormethan.

**24.** Verfahren gemäß Anspruch 22 oder 23, wobei das Wasser entionisiertes Wasser ist.

**25.** Verfahren gemäß einem der Ansprüche 19 bis 24, wobei das zweite, wasserfreie Lösungsmittel ausgewählt ist aus der Gruppe bestehend aus Methanol, Ethanol, Isopropylalkohol, Dimethylsulfoxid, Aceton und Tetrahydrofuran.

**26.** Verfahren gemäß einem der Ansprüche 19 bis 25, ferner umfassend das Mischen des Keratingemischs mit einem Aktivierungsmittel, ausgewählt aus der Gruppe bestehend aus einem Katalysator und einem Initiator.

**27.** Verfahren gemäß einem der Ansprüche 19 bis 26, wobei die Bedingungen das Exponieren des keratinbeschichteten Bindungsbereichs an eine Energiequelle über einen Zeitraum, der zum Herstellen des bioaktiven Bereichs wirksam ist, umfassen.

**28.** Verfahren gemäß Anspruch 27, wobei die Bedingungen das Vorhandensein des Aktivierungsmittels umfassen.

**29.** Verfahren gemäß Anspruch 27 oder 28, wobei der Zeitraum etwa 1 bis etwa 24 Stunden beträgt.

**30.** Verfahren gemäß Anspruch 29, wobei der Zeitraum etwa 24 Stunden beträgt.

**Revendications**

**1.** Dispositif médical comprenant :

un substrat comprenant un revêtement de passivation comprenant de la kératine, ledit revêtement de passivation comprenant une région de liaison et une région bioactive ;
ladite région de liaison comprenant au moins un composé organosilane comprenant un composant silane lié

à une surface dudit substrat ; et
ladite région bioactive comprenant un composant organique dudit organosilane lié à un groupe pendant réactif de ladite kératine.

2. Dispositif médical selon la revendication 1, dans lequel ledit composant silane dudit composé organosilane est lié de manière covalente à une surface du substrat.

3. Dispositif médical selon la revendication 2, dans lequel ladite région bioactive comprend des groupes pendants réactifs de ladite kératine liés de manière covalente audit composant organique dudit organosilane.

4. Dispositif médical selon la revendication 1, dans lequel ledit revêtement de passivation est efficace pour augmenter la formation de matrice osseuse assurée par des cellules ostéoblastes de souris 2T3 de culture.

5. Dispositif médical selon l'une quelconque des revendications précédentes, choisi dans le groupe constitué par les constructions d'ingénierie tissulaire, les implants orthopédiques, les implants médicaux, les implants dentaires et les dispositifs d'assistance ventriculaire.

6. Dispositif médical selon l'une quelconque des revendications précédentes, dans lequel ledit substrat comprend un matériau biocompatible.

7. Dispositif médical selon la revendication 6, dans lequel le matériau biocompatible est choisi dans le groupe constitué par le silicium, les métaux, les alliages de métaux et les céramiques.

8. Dispositif médical selon la revendication 6, dans lequel ledit matériau biocompatible est choisi dans le groupe constitué par le titane et l'hydroxyapatite.

9. Dispositif médical selon l'une quelconque des revendications précédentes, dans lequel ledit revêtement de passivation comprend de la kératine et un ou plusieurs facteurs bioactifs choisis dans le groupe constitué par la protéine morphogénique osseuse (BMP) et le facteur de croissance transformant bêta (TGF-$\beta$).

10. Dispositif médical selon l'une quelconque des revendications précédentes, dans laquelle ladite kératine est dérivée d'une substance choisie dans le groupe constitué par les cheveux, les poils, les plumes, les cornes, les sabots, les becs et les pieds.

11. Dispositif médical selon la revendication 10, dans lequel ladite kératine est de la kératine de cheveu humain.

12. Dispositif médical selon l'une quelconque des revendications précédentes, dans lequel ladite kératine comprend de la kératine réduite.

13. Dispositif médical selon l'une quelconque des revendications précédentes, dans lequel ladite kératine comprend de la kératine de masse moléculaire élevée (HMWK) ayant une masse moléculaire comprise entre environ 50 et environ 85 kDa.

14. Dispositif médical selon l'une quelconque des revendications précédentes, dans lequel ledit revêtement de passivation est efficace pour accélérer la formation de matrice osseuse par des ostéoblastes de souris 2T3 de culture.

15. Dispositif médical selon l'une quelconque des revendications précédentes, dans lequel ledit composant organique dudit organosilane comprend une fraction choisie dans le groupe constitué par les groupes époxy, les groupes alcoxy, les groupes vinyle, les groupes amine, les groupes isocyanate et les groupes carboxyle.

16. Dispositif médical selon la revendication 15, dans lequel lesdits groupes amine sont des groupes alkylamine.

17. Dispositif médical selon l'une quelconque des revendications précédentes, dans lequel ledit organosilane comprend des substituants choisis dans le groupe constitué par environ 1 à 3 atomes d'halogène et environ 1 à 3 groupes alcoxy.

18. Procédé de revêtement d'un dispositif médical avec un revêtement de passivation, ledit procédé comprenant les étapes consistant à :

lier un agent de couplage à une ou plusieurs surfaces dudit dispositif médical, produire une région de liaison ; et
lier la kératine à ladite région de liaison.

**19.** Procédé selon la revendication 18, dans lequel la liaison de la kératine à ladite région de liaison comprend les étapes consistant à :

dissoudre la kératine dans un solvant ; et
ajouter un second solvant anhydre pour produire un mélange de kératine ;
exposer ladite région de liaison audit mélange de kératine, produisant une région de liaison revêtue de kératine ; et
faire durcir ladite région de liaison revêtue de kératine dans des conditions efficaces pour produire ladite région bioactive.

**20.** Procédé selon la revendication 19, comprenant en outre le nettoyage de ladite ou desdites surfaces avant la liaison dudit agent de couplage à ladite ou auxdites surfaces.

**21.** Procédé selon la revendication 19 ou 20, comprenant en outre l'oxydation de ladite ou desdites surfaces avant la liaison dudit agent de couplage à ladite ou auxdites surfaces.

**22.** Procédé selon la revendication 20, dans lequel ledit nettoyage comprend la sonification de ladite ou desdites surfaces dans un premier solvant anhydre et la sonification de ladite ou desdites surfaces dans de l'eau.

**23.** Procédé selon la revendication 22, dans lequel ledit premier solvant anhydre est choisi dans le groupe constitué par le méthanol, l'éthanol, l'alcool isopropylique, le diméthylsulfoxyde, l'acétone, le tétrahydrofurane et le dichlorométhane.

**24.** Procédé selon la revendication 22 ou 23, dans lequel ladite eau est de l'eau désionisée.

**25.** Procédé selon l'une quelconque des revendications 19 à 24, dans lequel ledit second solvant anhydre est choisi dans le groupe constitué par le méthanol, l'éthanol, l'alcool isopropylique, le diméthylsulfoxyde, l'acétone et le tétrahydrofurane.

**26.** Procédé selon l'une quelconque des revendications 19 à 25, comprenant en outre le fait de mélanger le mélange de kératine avec un activateur choisi dans le groupe constitué par un catalyseur et un initiateur.

**27.** Procédé selon l'une quelconque des revendications 19 à 26, dans lequel lesdites conditions comprennent l'exposition de ladite région de liaison revêtue de kératine à une source d'énergie pendant une durée efficace pour produire ladite région bioactive.

**28.** Procédé selon la revendication 27, dans lequel lesdites conditions comprennent la présence dudit activateur.

**29.** Procédé selon la revendication 27 ou 28, dans lequel ladite durée est d'environ 1 à environ 24 heures.

**30.** Procédé selon la revendication 29, dans lequel ladite durée est d'environ 24 heures.

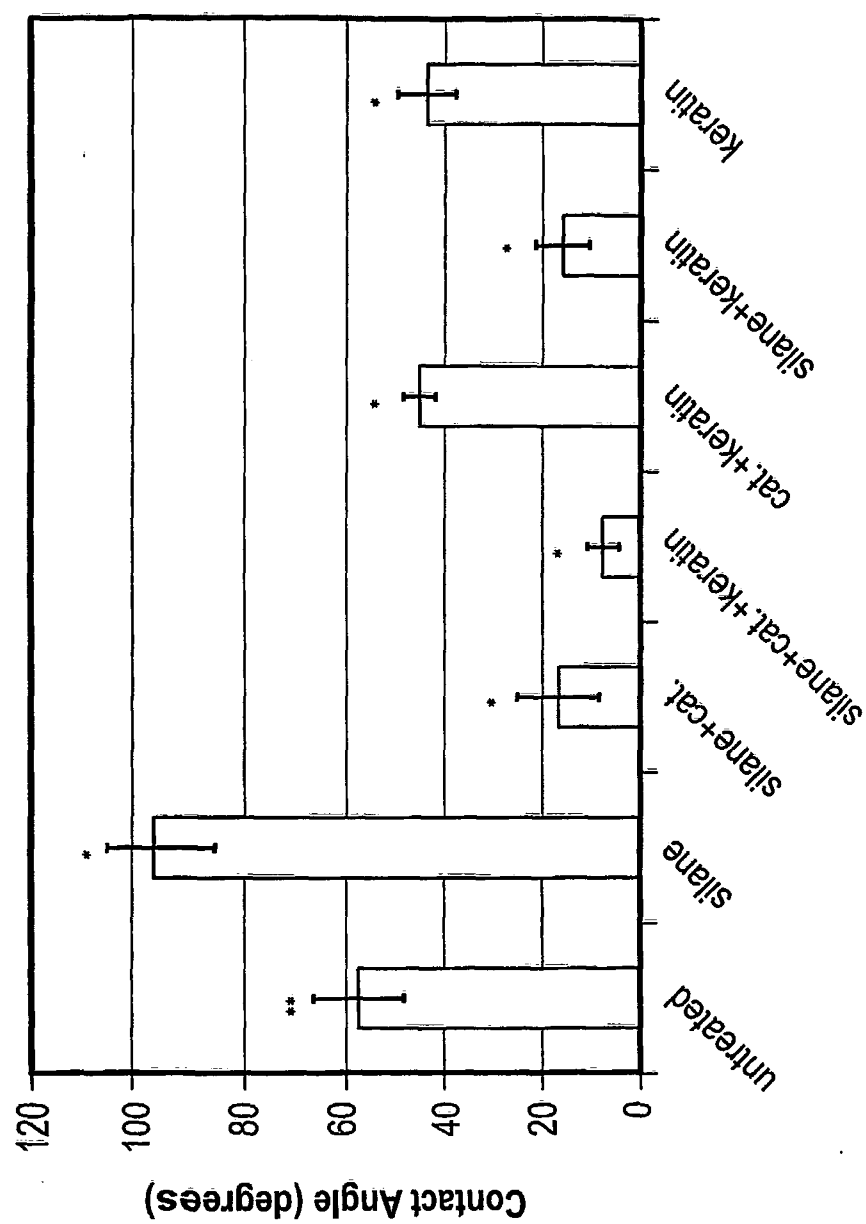
Contact Angle (degrees)
0
20
40
60
80
100
120
untreated
silane
silane+cat.
silane+cat.+keratin
cat.+keratin
silane+keratin
keratin

FIG. 1

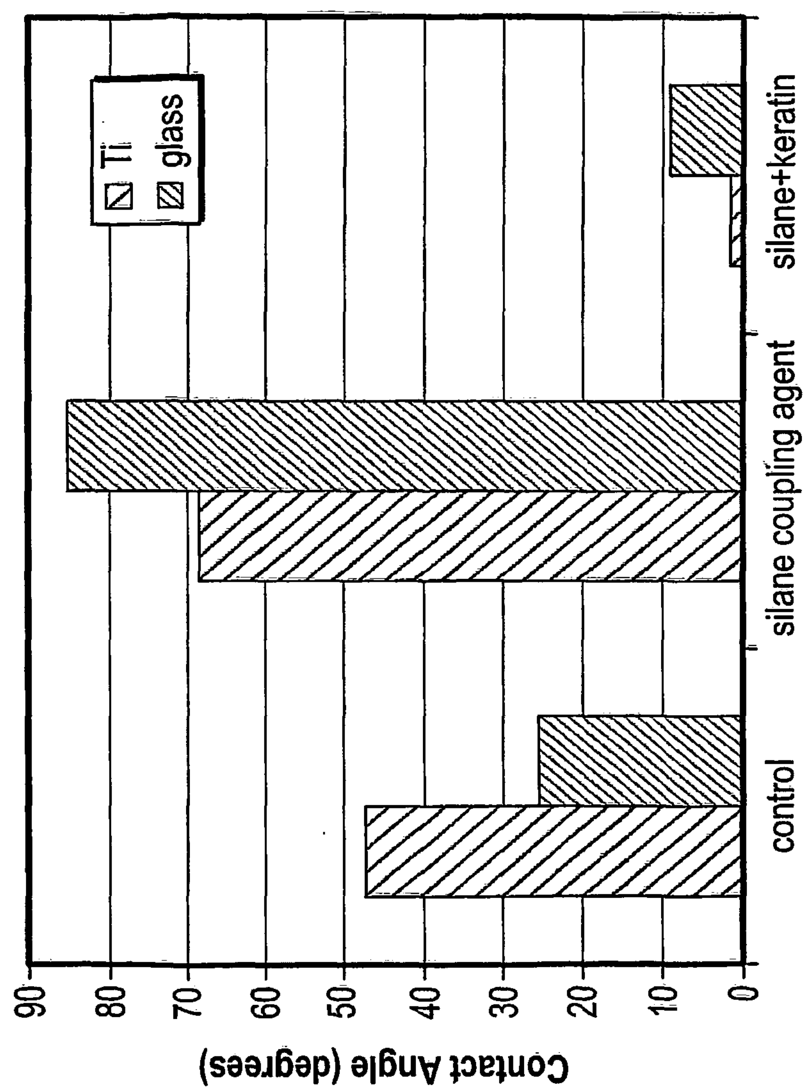
Contact Angle (degrees)
0
10
20
30
40
50
60
70
80
90
control
silane coupling agent
silane+keratin
Ti
glass

*FIG. 2*

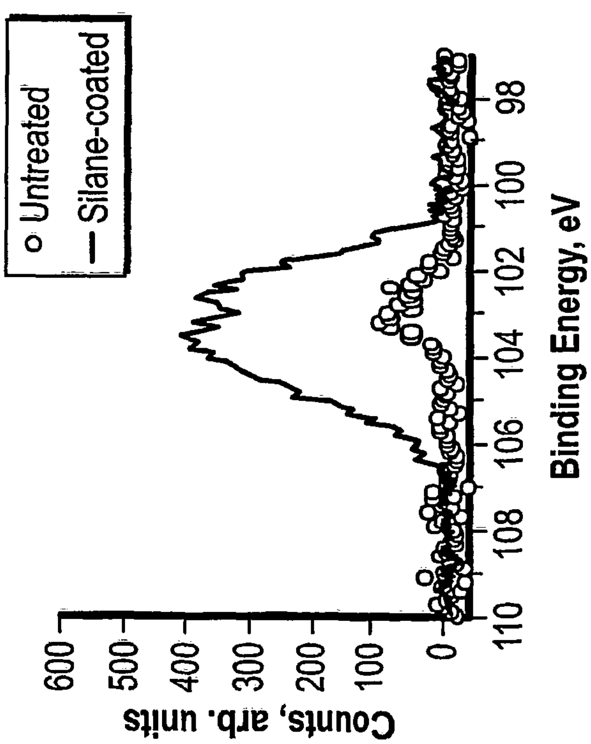
Untreated
Silane-coated
Counts, arb. units
600
500
400
300
200
100
0
110
108
106
104
102
100
98
Binding Energy, eV

FIG. 3A

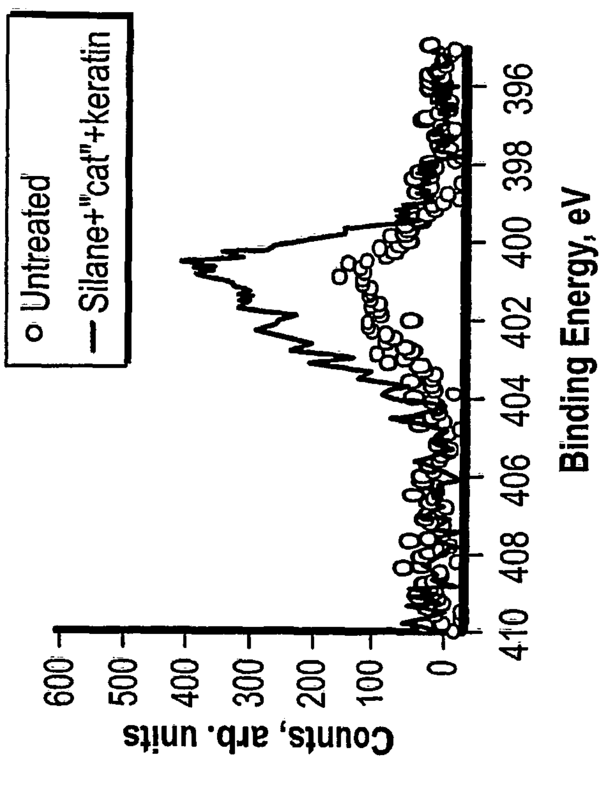
Untreated
Silane+"cat"+keratin
Counts, arb. units
600
500
400
300
200
100
0
410
408
406
404
402
400
398
396
Binding Energy, eV

FIG. 3B

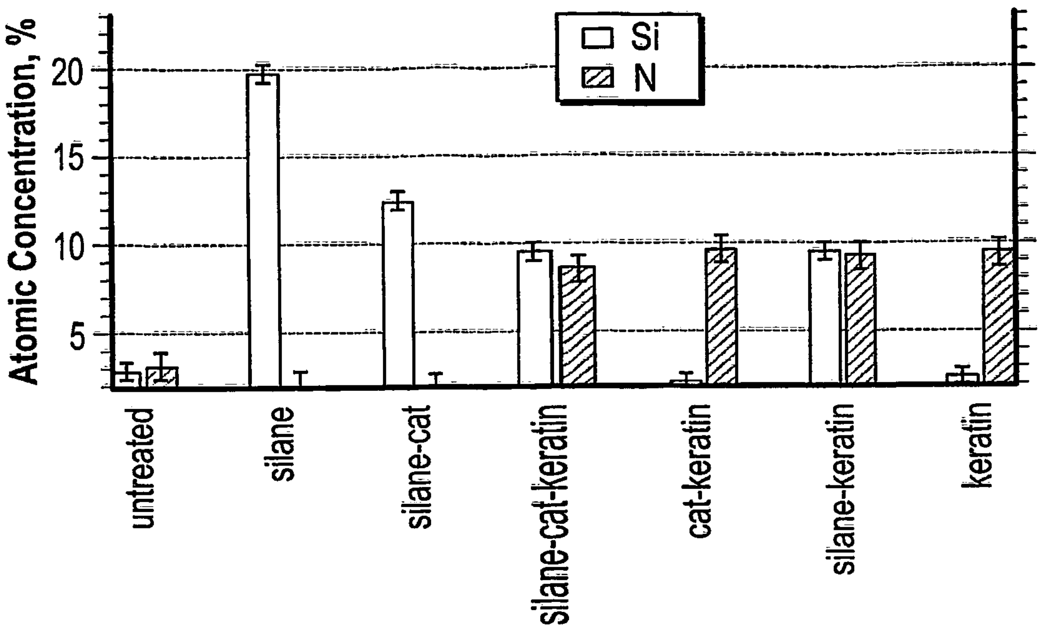
Si
N
Atomic Concentration, %
20
15
10
5
untreated
silane
silane-cat
silane-cat-keratin
cat-keratin
silane-keratin
keratin

*FIG. 4*

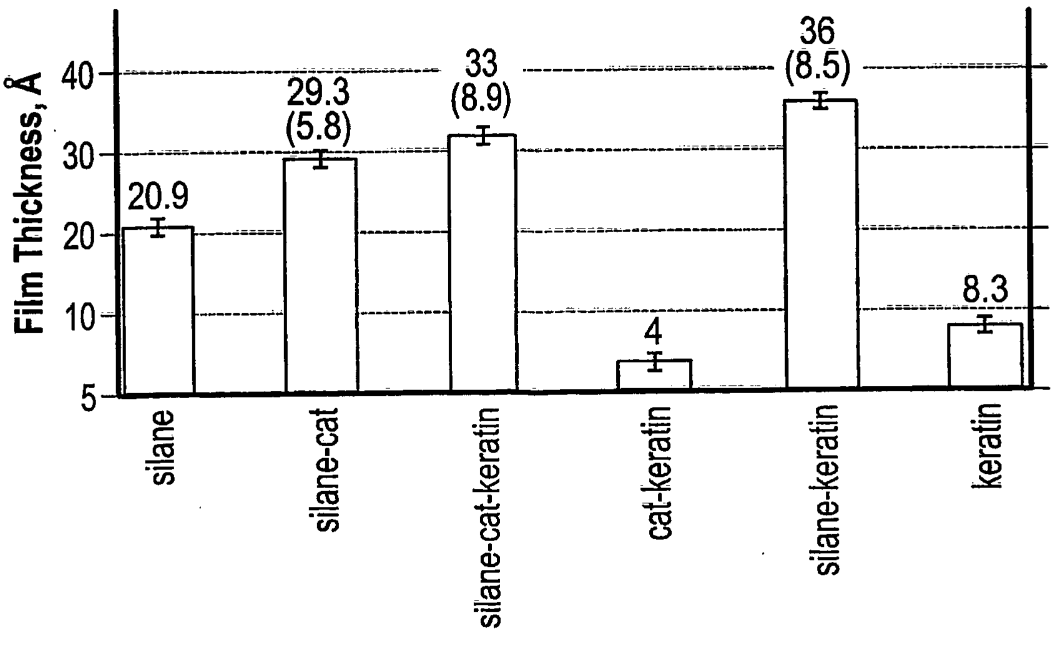
Film Thickness, Å
40
30
20
10
5
20.9
29.3
(5.8)
33
(8.9)
4
36
(8.5)
8.3
silane
silane-cat
silane-cat-keratin
cat-keratin
silane-keratin
keratin

*FIG. 5*

AsA/GP

\+ 

−

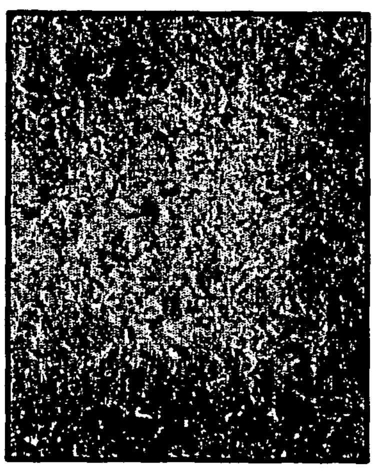

FIG. 6A

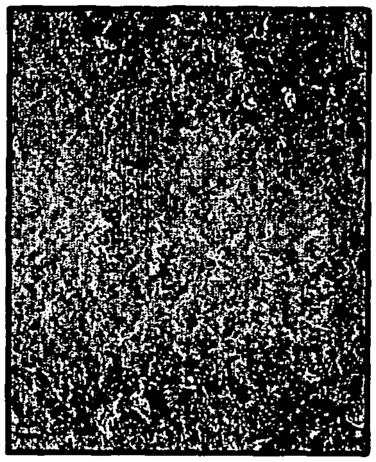

FIG. 6B

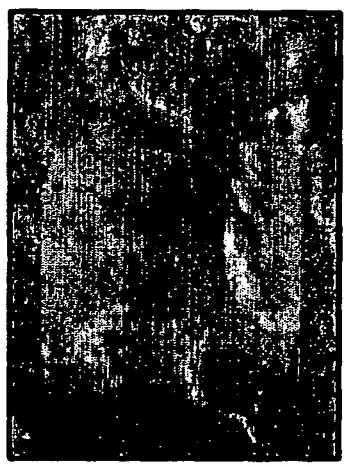

FIG. 6C

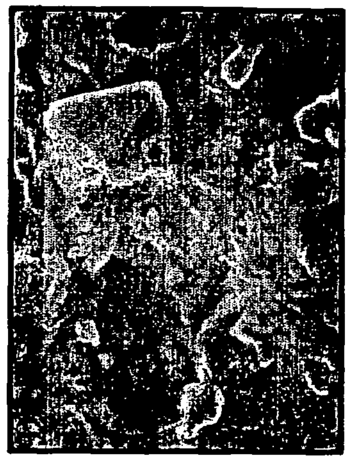

FIG. 6D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- the Chemistry of the Thiol Group. John Wiley & Sons, 1974
- **REMPP, P. ; MERRILL, E. W.** Polymer Synthesis. Huethig & Wepf Verlag, 1986
- **YOUNG, R. J. ; LOVELL, P. A.** Introduction to Polymers. Chapman & Hall, 1991
- **ODIAN, G.** Principles of Polymerization. John Wiley & Sons, 1991
- Preparation of Thiols. **WARDELL, J. L.** The Chemistry of the Thiol Group. John Wiley & Sons, 1974, 163-353
- The Chemistry of Keratins. **CREWTHER, W. G. ; FRASER, R. D. B. ; LENNOX, F. G. ; LINDLEY, H.** Advances in Protein Chemistry. Academic Press, 1965, 191-346
- **ARKELS B.** Tailoring surfaces with silanes. *Chemtech,* 1977, vol. 7, 766-778
- **C. S. FADLEY.** *Progress in Surface Science,* 1984, vol. 16, 275-388
- **GHOSH-CHOUDHURY N ; WINDLE JJ ; KOOP BA ; HARRIS MA ; GUERRERO, DL ; WOZNEY JM ; MUNDY GR ; HARRIS SE.** Immortalized murine osteoblasts derived from BMP2-T-anigen expressing transgenic mice. *Endocrinology,* 1996, vol. 137, 331-339
- **BERESFORD JN ; GRAVES SE ; SMOOTHY CA.** Formation of mineralized nodules by bone derived cells in vitro: a model of bone formation?. *Am. J. Med. Genet.,* 1993, vol. 45, 163-78